# EUROPEAN PATENT APPLICATION

(11) **EP 2 319 522 A1**
(43) Date of publication of application: **11.05.2011**
(21) Application number: 10190094.2
(22) Date of filing: 05.11.2010
(51) Int. Cl.: A61K 36/752, A61K 36/70, A61P 13/04

(54) **An aqueous solution for treating nefrolithiasis comprising active ingredients from citrus fruits of the genus Citrus (Citrus limonum, Citrus aurantifolia) in the family Rutaceae and of the leaves of seagrape (Coccoloba uvifera).**

(30) Priority: 06.11.2009 IT CZ20090024
(71) Applicant: Cianni, Vincenzo, 87020 Sangineto (CS) (IT)
(72) Inventor: Cianni, Vincenzo, 87020 Sangineto (CS) (IT)
(74) Representative: Comoglio, Elena

(57) **Abstract**

The invention relates to an aqueous solution for treating nefrolithiasis comprising a synergistic combination of active ingredients extracted from fruits of citrus genus (Citrus limonum, Citrus aurantifolia), of the Rutaceae family, and from the leaves of seagrape (Coccoloba uvifera), preferably from spontaneous plants capable of producing seagrape.

## Description

The subject matter of the present invention is an aqueous solution for treating nefrolithiasis.

More particularly, the subject matter of the invention is an aqueous solution of active ingredients extracted from two plants, a citrus fruit of the genus citrus (Citrus limonum, Citrus aurantifolia), of the Rutaceae family, and seagrape (Coccoloba uvifera).

Citrus limonum, or lemon, is a fruit tree belonging to the Rutaceae family. Lemon, native of the Far East, is an invaluable natural remedy and can be considered a sort of universal panacea thanks to the combination of the precious active ingredients it contains.

Lemon fruits are particularly rich in vitamin C (100 g of lemon contain 40-50 mg; vitamin C is known to be essential to life, to be a fundamental antioxidant (acting against free radicals and on the endocrine glands); in B group vitamins (which are important for nervous equilibrium and for nourishment and equilibrium of the skin); in vitamin A (which is important for keeping tissues young and for growth); in vitamin PP (which protects blood vessels). Lemon also contains mineral salts and trace elements (of which calcium, iron, silica, phosphorus, manganese and copper), bioflavonoids (which are responsible for its characteristic yellow colour), citric acid, calcium citrate and potassium citrate (natural acidity regulators) and essential oils (mainly present in the rind). The soluble sugars contained in lemon, i.e. glucose and fructose, are directly assimilable. It also contains gums, mucilages, albumins, terpenes, linalol, aldehydes and lemon camphor.

Thanks to the natural substances it contains, lemon is a natural alkalizing agent, gastric antiacid, antiseptic, bactericide, activator of white blood cells (immune defences), mild anti-infiammatory, tonic for the sympathetic and nervous system, cardiac tonic, detoxicant, depurative and diuretic, antirheumatic, antigout, antiarthritic, antischlerotic (combats ageing), anti scorbutic and antivenom, blood-fluidizer, hypotensive (lowers blood pressure), remineralizer, antianemic, gastro-hepatic-pancreatic stimulator, haemostat, carminative and vermifuge, antipruritic, tonic and antidiarrhoeal.

Similar considerations can be made about Citrus aurantifolia, or lime. Lime is a fruit of the gender Citrus, also of the Rutaceae family. It is probably a hybrid between citron and lemon, but the experts do not agree on its origin. The prevailing opinion is that lime comes from repeated hybridizations among various Citrus and perhaps with kumquat (Chinese tangerine) too, until obtaining this fruit whose peculiar and lasting characteristics enable us to regard it as a separate species of the gender Citrus.

Lime trees are mainly grown to produce essential oil from its rind. Its essence is very similar to the lemon's, and is very appreciated in the perfume and detergent industry, although the main quantity is set aside for alcohol-free drinks production.

There are also many lime fruit plantations that grow only ornamental trees, because this evergreen, like all the trees of the gender Citrus, in favourable conditions flowers all year long. Its small coloured fruits are very picturesque, chiefly because they stay long on the branches together with the flowers of the following harvest.

Seagrape (Coccoloba uvifera) is a medium-sized plant that can reach two meters in height; in summer it turns white. It is an evergreen, and, as such, it retains its leaves all year long. Coccoloba uvifera develops as a shrub. It is grown in direct sunlight. Seagrape suffers cold temperatures, consequently, in spring, it should be exposed in open air only when temperatures are above 15°C. To prevent cold weather damages, during the months in which temperatures are particularly harsh, shrubs are protected by covering the ground around them with straw or dry leaves.

Coccoloba uvifera is already known in literature for being used as a medicament. In particular, both the juice and the decoctions of its wood, its bark and its roots are used to treat diarrohea. Besides, other parts of this plant are ingested for treating dysentery, haemorrhage and venereal diseases, and are used externally for treating skin problems. Infusions of Coccoloba uvifera leaves are also used for treating hoarseness and asthma and for dressing wounds.

There is a patent application No. CA2313879 of April 21st 2001 relating to a method for controlling sugar levels in blood using Coccoloba uvifera. This method enables us to continuously monitor the variations of the quantity of glucose in blood.

Nevertheless, neither the state of the art currently in use nor the patent applications above mentioned describe similar inventions and/or methods that overcome the critical aspects mentioned. The inventor has noticed that the innovative combination of plants of the genus Citrus (Citrus limonum, Citrus aurantifolia), of the Rutaceae family, with seagrape, and of the active ingredients they contain - in particular, the active ingredients present in lemon fruits or lime fruits and in seagrape leaves, that have precious complementary and synergistic properties - favour in a natural way the physiologic functions of the whole body.

Thanks to this observation, the inventor has produced an aqueous solution for treating nefrolithiasis, comprising a synergistic combination of active ingredients extracted from fruits of the genus citrus (Citrus limonum, Citrus aurantifolia), of the Rutaceae family, and from seagrape (Coccoloba uvifera) leaves. For such purpose, the use of spontaneous plants is to be preferred. The aqueous solution of the invention has revealed particularly effective as an alkalizing agent, depurative, detoxicant, tonic, stimulator of the internal organs activity and revitalizer of the whole body, since it re-equilibrates its metabolism. It has also proven to have beneficial effects on blood, making it more fluid, as well has having cooling, mineralizing and vitaminizing properties.

Such applications make the solution of the present invention an extremely effective remedy in a range of applications concerning the well-being of the body; in addition, it has an unusual property which distinguishes it from all the other aqueous solutions: its alkaline nature.

Actually, being the aqueous solution of the invention made from lemon juice, it is also useful for reintegrating a diet with a low alkaline content (fruit and vegetables) or in balancing diets with high protein or fat content which would otherwise have an excessively acid content for the body.

Such condition, if protracted, could facilitate the onset of some diseases, such as osteoporosis and nefrolithiasis, which manifest themselves precisely by low levels of citrates in urine.

Advantageously, the Coccoloba uvifera-based solution, which is the subject matter of the patent application, is strongly indicated for nefrolithiasis treatment, even being capable of dissolving kidney stones.

Furthermore, its curative action occurs against menstrual pain, against the prostate-related problems and the affections of the respiratry system, such as, in particular, bronchitis. Its action as powerful diuretic has been also noticed.

The solution of the invention is prepared by a decoction process. Preservatives or stabilizers that do not alter in the least the properties of the solution can be added.

The following example is provided for illustrative purposes, without the intent of limiting the scope of the invention as defined in the appended claims.

About 8 kg of lemon or lime fruits, complete with rind, are cut in halves and placed into a boiler with about 70 litres of water and 10 kg of seagrape leaves from wild plants producing seagrape. The mixture is made to boil for about 10-12 hours. The boiling for such a long time determines both the extraction of the active ingredients contained in lemon fruits and in the wild seagrape leaves, and the reduction of the volume to about 30 litres, with consequent concentration of the active ingredients extracted. At this point the mixture obtained is filtered and bottled.

The solution can be used as a remedy against the disorders listed above with a cycle of treatment consisting in taking 80-85 ml of product twice a day in the morning on an empty stomach and in the evening before going to bed, for 3 consecutive days. The use of the solution is not recommended during pregnancy, breast-feeding and in treating children aged less than three.

It is understood that the subject-matter of the present invention is not limited to the description given, and can be improved and modified by those of ordinary skill in the art without exceeding the scope of the patent.

## Claims

1. An aqueous solution for treating nefrolithiasis, **characterized in that** it comprises a synergistic combination of active ingredients extracted from fruits of citrus genus (Citrus limonum, Citrus aurantifolia), of the Rutaceae family, and from the leaves of seagrape (Coccoloba uvifera).

2. The aqueous solution for treating nefrolithiasis, according to claim 1, **characterized in that** the leaves of seagrape (Coccoloba uvifera) are obtained from spontaneous plants capable of producing seagrape.

3. The aqueous solution for treating nefrolithiasis, according to claim 1, **characterized in that** preservatives or stabilizers are present.

4. The aqueous solution for treating nefrolithiasis, according to claim 1, **characterized in that** extraction is made by decoction.
